# EUROPEAN PATENT SPECIFICATION

(11) **EP 2 695 611 B1**
(45) Date of publication and mention of the grant of the patent: **01.10.2014**
(21) Application number: 12179384.8
(22) Date of filing: 06.08.2012
(51) Int. Cl.: A61K 31/167, A61K 9/00, A61K 8/42, A61Q 7/00, A61P 17/14

(54) **Eprotirome for use in the prevention and/or treatment of hair disorders and compositions thereof**
Eprotirom zur Verwendung bei der Vorbeugung und/oder Behandlung von Haarerkrankungen und Zusammensetzungen davon
Eprotirome pour l'utilisation dans la prévention et/ou le traitement de troubles de cheveux et compositions correspondantes

(43) Date of publication of application: 12.02.2014
(73) Proprietor: Dr. August Wolff GmbH & Co. KG Arzneimittel, 33611 Bielefeld (DE)
(72) Inventor: Soeberdt, Michael, 33605 Bielefeld (DE); Knie, Ulrich, 32105 Bad Salzuflen (DE); Abels, Christoph, 33619 Bielefeld (DE)
(74) Representative: Ter Meer Steinmeister & Partner

(56) References cited:
- EP-A2- 1 262 177
- WO-A1-00/72812
- WO-A1-01/60784
- ANGELIN B ET AL: "Abstract: 1463 EPROTIROME: A PHYSIOLOGICAL APPROACH TO LIPID CONTROL UTILIZING A LIVER SELECTIVE THYROID HORMONE RECEPTOR AGONIST", ATHEROSCLEROSIS SUPPLEMENTS, ELSEVIER, AMSTERDAM, NL, vol. 10, no. 2, 1 June 2009 (2009-06-01), page e443, XP026780704, ISSN: 1567-5688, DOI: 10.1016/S1567-5688(09)70434-9 [retrieved on 2009-06-01]

## Description

The present invention provides methods for preventing and/or treating hair disorders, in particular for preventing and/or treating hair graying and/or hair loss, including arresting and/or reversing hair loss and promoting hair growth, in a mammal. Moreover, the present invention relates to eprotirome as well as pharmaceutical compositions containing eprotirome and pharmaceutically acceptable excipients for use in preventing and/or treating hair disorders.

### Background of the Invention

Hair loss is a common problem not only in men but also in women. Hair loss occurs for example due to physiological or pathological processes or is promoted by drugs, e.g. retinoids, chemotherapeutic agents, cholesterol lowering agents etc., used in indications such as cancer. In many cases patients do not only suffer from hair loss, but there is also a lack of hair regrowth. Both defects can lead to partial or full baldness.

It is well known that hair grows in cycles including different phases. The main phases are the growth phase (anagen), the involuting or regressing phase (catagen) and the resting or quiescent phase (telegon). During anagen the root of the hair is dividing rapidly, leading to hair shaft elongation. The rather short catagen phase is a transition stage in which the end of active growth of a hair is signaled. Finally, telogen is the resting phase in which hair is shed.

Commercially used therapeutic agents for the treatment of hair losss are minoxidil (Rogain^{®}), a potassium channel opener, and finasteride (Propecia®), a 5α-reductase inhibitor. However, these agents are only of limited effectiveness.

Moreover, it has been shown in literature that thyroid hormone receptor agonists are linked with hair growth. Two natural thyroid hormones are known, namely thyroxine or 3,5,3',5'-tetraiodo-L-thyronine (T4) and thyronine or 3,5,3'-triiodo-L-thyronine (T3). T3 is the biological more active form. It differs from T4 by the absence of the 5' iodine. It can be directly produced from the thyroid gland or can be obtained by deiodination of T4 with the help of two deiodinase enzymes.

The use of T3 or T4 for stimulating or enhancing hair growth has been claimed in WO96/25943. Thyroid deficiency related hair abnormalities have been known for a long time (Messenger, Br. J. Dermatol. 2000, 142: 633-634). Thyroid hormone nuclear receptors (TR) have been localized in human hair follicles. Immunoreactive TR were detected in the nuclei of the outer root sheath cells, dermal papilla cells, and fibrous sheath cells of hair follicles. Treatment of these cell types with T3 stimulated the proliferation and/or metabolism of all these types of cells significantly (Ahsan et al., J. Med. Invest., 1998, 44: 179-184). A subsequent study revealed that mainly thyroid hormone receptor β1 (TRβ1) is expressed in human hair follicles (Billoni et al., Br. J. Dermatol., 2000, 142: 645-652) and that a physiological level of free T3 significantly enhanced human hair survival *in vitro* in a hair follicle culture model. *In vivo*, topical T3 was shown to stimulate epidermal proliferation, dermal thickening, and hair growth in SKH-1 mice and CD rats dramatically, offering a new strategy for treating skin and hair disorders (Safer et al., Thyroid, 2001, 11: 717-724). In a more recent study it was shown that both T3 and T4 prolong anagen duration in a human hair organ culture model (van Beek et al., J. Clin. Endocrinol. Metab., 2008, 93: 4381-4388). This histomorphometrical finding, however, did not translate to a significantly altered hair shaft elongation *in vitro.* As shown with Ki-67/TUNEL staining T4 stimulated hair matrix keratinocyte proliferation, whereas both T3 and T4 inhibited apoptosis of these cells. Furthermore, T3 and T4 were able to stimulate melanin synthesis in human hair follicles.

Sulfonyl thyromimetic compounds for treating hair loss are claimed in WO 00/72810. WO 00/72811 discloses methods of treating hair loss using certain compounds, such as substituted phenoxybenzoic acids, described therein. Methods of treating hair loss using certain diphenylether derivatives are disclosed in WO 00/72812. WO_00/72813 discloses methods of treating hair loss using certain diphenylmethane derivatives. Substituted biaryl ether compounds and compositions for treating hair loss are disclosed in WO 00/72920. WO 00/73292 discloses biaryl compounds and compositions for treating hair loss.

Indole carboxylic acids as thyroid receptor ligands are described in EP 1297833. The compounds are claimed to be useful in the treatment of hair loss. EP 1262177 discloses the medical use of thyromimetic compounds to treat hair loss and compositions. WO 01/072692 describes the preparation of N-phenylmalonamic acid derivatives with thyroid receptor ligand activity. The compounds are claimed to be useful for the treatment of inter alia alopecia. WO 03/064369 describes the preparation of indane derivatives as thyroid hormone receptor ligands. WO 08/001959 discloses the preparation of 6-5 bicyclic heterocyclic derivatives as thyroid hormone receptor ligands. The title compounds are inter alia claimed for the treatment of alopecia. JP 2009155261 describes indole compounds and pharmaceutical compositions comprising these compounds for the treatment of diseases through thyroid hormone receptor-mediated control of cell functions.

The use of cardiac-sparing TR agonists for treatment of hair loss has been claimed in EP 1262177 B1. According to the patent the claimed thyromimetic compounds can be used in a topical formulation for treating hair loss, in particular male and female pattern baldness. One of the claimed substances (PF277343) has been tested in mouse and monkey hair growth models and was found to be efficacious in both species (Li et al., Bioorg. Med. Chem. Lett., 2010, 20: 306-308).

WO 01/060784 claims aniline-derived ligands for the thyroid receptor. Use of the compounds for treating obesity, hypercholesterolemia, atherosclerosis, depression, osteoporosis, hypothyroidism, goiter, thyroid cancer, glaucoma, cardiac arrhythmia, congestive heart failure, or a skin disorder or disease is claimed. The skin disorder or disease is dermal atrophy, post surgical bruising caused by laser resurfacing, keloids, stria, cellulite, roughened skin, actinic skin damage, lichen planus, ichtyosis, acne, psoriasis, Darier's disease, eczema, atopic dermatitis, chloracne, pityriasis and skin scarring.

WO 03/039456 claims a process for the preparation of aniline-derived thyroid receptor ligands with improved safety and economy. WO 07/11025 claims improved crystalline material. WO 07/11026 claims stable oral pharmaceutical composition containing thyroid hormone receptor agonists. WO 07/110225 describes pharmaceutical compositions containing novel crystal forms of 3-[[3,5-dibromo-4-[4-hydroxy-3-(1-methylethyl)-phenoxy]-phenyl]-amino]-3-oxopropanoic acid. WO 09/077147 discloses pharmaceutical compositions of thyroid hormone receptor-binding compounds. WO 09/080835 claims thyromimetic compounds in treatment of disease related to sonic hedgehog signalling.

Thus, some thyroid hormones and the above-mentioned TR agonists are claimed to be efficacious in treating hair loss. However, all of these therapeutic agents have either been shown to cause severe side effects or not to be effective for a serious treatment of hair loss.

In order to treat hair loss, the systemic administration of T3 and/or T4 is not practicable because these thyroid hormones are known to cause adverse side effects, such as inducing significant cardiotoxicity or adversely affecting bone mineral density and lean body weight. Very recently, results of a randomized, double-blind pilot clinical efficacy trial with topical T3 were published (Nasiri et al., JEADV, 2011, DOI: 10.1111/j.1468-3083.2011.04088.x). In this first clinical study with topical T3 in alopecia areata the compound was found to be safe but not more effective than placebo. Further, also the use of TR agonists has not been shown to result in sufficient therapeutic efficiency while at the same time being applied in a safe manner.

Hence, there is a strong demand for an effective and safe long-term treatment for inducing hair growth or preventing hair loss. In particular, new therapeutic agents are necessary, which are both highly effective in the treatment and/or prevention of hair loss and which do not show the above-mentioned side effects, such as cardiotoxicity, bone defects or the loss of body weight.

In view of the unresolved deficiencies in treating hair loss as discussed above, it is an object of the present invention to provide an effective and safe long-term treatment for inducing hair growth or preventing hair loss which does not show the above-mentioned side effects.

The above-mentioned problems have surprisingly been solved by the inventors of the present invention in that they found out that the eprotirome (KB2115, 3-[[3,5-dibromo-4-[4-hydroxy-3-(1-methylethyl)phenoxy]phenyl]amino]-3-oxo-propanoic acid) is able to promote hair growth and to exhibit pigmentation-stimulatory effects while at the same time does not cause side effects, such as inducing significant cardiotoxicity or adversely affecting bone mineral density and lean body weight.

In particular, the inventors of the present invention found out that eprotirome clearly prolongs anagen as seen in hair staging histomorphometry following *in vitro* testing in a human hair follicle organ culture and confirmed by an increased proliferation of hair matrix keratinocytes and a significant reduced expression of TGFβ2. Furthermore, upregulation of tyrosinase activity and increased melanin content in treated hair follicles reflect pigmentation-stimulatory effects.

### Summary of the Invention

Therefore, the subject-matter of the present invention according to claim 1 is eprotirome for use in the prevention and/or treatment of hair loss in a mammal.

Further, the subject-matter of the present invention is also a pharmaceutical composition for use in the prevention and/or treatment of hair loss in a mammal comprising:
(a) eprotirome as active ingredient, and
(b) a pharmaceutically acceptable excipient.

Consequently, the present invention relates to eprotirome and to a pharmaceutical composition comprising eprotirome as active ingredient and pharmaceutically acceptable excipients for use in the prevention and/or treatment of hair loss, including arresting and/or reversing hair loss and promoting hair growth.

Finally, the present invention relates to the non-therapeutic use of eprotirome as defined in claim 14.

### Detailed description of the invention

The chemical name of eprotirome, also known as KB2115, is 3-[[3,5-dibromo-4-[4-hydroxy-3-(1-methylethyl)phenoxy]phenyl]amino]-3-oxo-propanoic acid). The chemical structure of eprotirome is illustrated in the following:

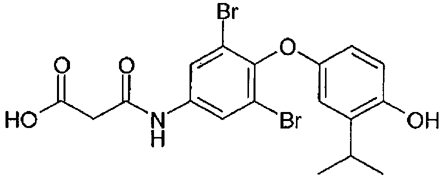

So far, eprotirome has been found to be effective on cholesterol levels in patients suffering from overweight. In a clinical trial in humans the TR-β selective thyromimetic eprotirome was found to be safe and well tolerated and did not provoke detectable effects on the heart while total and LDL cholesterol levels in moderately overweight and hypercholesterolemic subjects were lowered up to 40% (Berkenstam et al., PNAS, 2008, 105: 663-667).

TRβ and TRα are widely expressed and have distinct patterns of expression (Forrest et al., EMBO J. 1996, 15: 3006-3015). In humans an especially high expression of TRα1 is found in cardiac (Blange et al., C. Bol. Pharm. Bull., 1997, 20: 1123) and skeletal muscles. TR-β1 is predominately expressed in brain, liver and kidney.

The present invention relates to eprotirome for use in the prevention and/or treatment of hair loss in a mammal.

In the present invention the term eprotirome is understood in the sense of all active forms of eprotirome, including, for example, the free form thereof, e.g., the free acid form, and also, polymorphs, hydrates, solvates, tautomers, and all pharmaceutically acceptable salts thereof, unless specifically stated otherwise. It is further understood that suitable active metabolites of eprotirome, in any suitable form, are also encompassed by the term eprotirome.

According to the invention eprotirome can be present in the form of pharmaceutical acceptable salts. The term "pharmaceutical acceptable salts" refers to salts prepared from pharmaceutically acceptable non-toxic bases including inorganic or organic bases. Salts derived from inorganic bases include aluminum, ammonium, calcium, copper, ferric, ferrous, lithium, magnesium, manganic salts, manganous, potassium, sodium, zinc and the like. Particularly preferred are the ammonium, calcium, lithium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

Eprotirome useful in the methods of the present invention is cardiac-sparing. The term "cardiac-sparing" as used herein means that, at dosages required for promoting hair growth and for preventing graying, eprotirome, useful in the methods of the present invention does not result in any observable cardiotoxicity in mammals being treated.

As such, eprotirome is expected to have a stronger effect on hair growth than on cardiac endpoints and other undesirable endpoints.

In the present invention an effective amount of eprotirome is preferably used for the prevention and/or treatment of hair disorders in a mammal.

As used herein, "effective amount of eprotirome" means an amount that is effective to exhibit biological activity, preferably wherein the biological activity is arresting and/or reversing hair loss or promoting hair growth and/or preventing graying, at the site(s) of activity in a mammalian subject, without undue adverse side effects (such as undue toxicity, irritation or allergic response), commensurate with a reasonable benefit/risk ratio when used in the manner of the present invention.

The "effective amount of eprotirome" is typically from about 0.01 mg to 1000 mg, more preferably from 0.05 mg to 100 mg, most preferably from 0.1 to 10 mg of eprotirome administered per day.

According to the present invention, a mammal is understood to be a member of the class Mammalia, air-breathing vertebrate animals characterised by the possession of endothermy, hair, three middle ear bones, and mammary glands functional in mothers with young. A preferred mammal is a human being.

In the present invention, according to claim 1, the hair disorder is hair loss, including arresting and/or reversing hair loss and promoting hair growth.

In particular, hair loss is preferably selected from alopecia areata, androgenetic alopecia including male pattern and female pattern baldness and chemotherapy-induced hair loss.

It is understood that eprotirome may be used in the treatment of conditions such as treating hair loss in mammals, including arresting and/or reversing hair loss and promoting hair growth. Such conditions may manifest themselves in, for example, telogen effluvium, postpubertal effluvium, senile effluvium, alopecia areata and androgenetic alopecia including male pattern baldness and female pattern baldness, dystrophic effluvium and alopecia areolaris specifica.

Eprotirome may also be used to accelerate the regrowth of hair following mechanical alopecia and episodal telegon effluvium induced by heavy blood loss, acute severe infectious diseases, shocks induced by surgeries and traumatic shocks, acute flare-ups of systemic diseases like collagenosis and medicaments including chemotherapy-induced hair loss. In addition eprotirome may have pigmentary effects, including the prevention or reversal of graying.

An alternative embodiment of the present invention according to claim 14 relates to the non-therapeutic (cosmetic) use of eprotirome in the treatment and/or prevention of hair loss in mammals and/or in the prevention of hair conditions, preferably in the prevention and/or reversal of hair graying.

In general, according to the present invention, eprotirome can be administered topically, enterally or parenterally. Preferably, eprotirome is administered topically.

Furthermore, the present invention relates to a pharmaceutical composition for use in the prevention and/or treatment of hair loss comprising:
(a) eprotirome according to the present invention as active ingredient , and
(b) a pharmaceutically acceptable excipient.

Preferably, the present invention relates to a pharmaceutical composition containing eprotirome as an active ingredient for use in the treatment and/or prevention of hair loss, and a pharmaceutically acceptable excipient.

Moreover, the present invention relates to the non-therapeutic use of a cosmetic composition comprising:
(a) eprotirome according to the present invention as active ingredient for use in the treatment and/or prevention of hair loss in mammals and/or in the prevention of hair conditions, preferably in the prevention and/or reversal of hair graying.
(b) a cosmetically acceptable excipient.

In the present invention, the composition is preferably a dermatological composition suitable to be applied topically on the skin of a mammal. The form of the composition is not particularly limited; In preferred embodiments, the compositions are in the form of lotions, creams, gels, sprays, powders, ointments, waxes, soaps, shampoos, hydroalcoholic solutions, emulsions, suspensions, solutions, foams, saturated pads, skin or hair conditioning agents. More preferred forms are emulsions, suspensions and solutions.

Particularly, eprotirome is incorporated into pharmaceutical or cosmetic preparations by admixing it with pharmaceutically/dermatologically acceptable excipients.

Compositions useful in accordance with the present invention may contain cosmetically and pharmaceutically/dermatologically acceptable excipients known to the skilled person. These include for example solvents such as organic solvents, gelling agents, buffers, detergents, oils, alcohols, emulsifiers, solubilizers, humectants, fillers, bioadhesives, emollients, preservatives, bactericides, surfactants, perfumes, thickeners, softening agents, moisturizing agents, oils, fats, waxes, water, alcohols, polyols, polymers, foam stabilizers, foaming agents, antifoaming agents, hair coating agents or other suitable components of a pharmaceutical or cosmetic preparation.

Surfactants can be chosen form the group of anionic, cationic, non-ionic and amphoteric surfactants. Anionic surfactants are for example alkylether sulfates, alkyl sulfates, alkyl-sulfosuccinates, ether carboxylic acids and ester carboxylic acids, alkylsacosinates, -glutamates, - glycinates and -taurates, alkylphosphates and alkylsulfonates. Examples for amphoteric surfactants are cocoamidopropyl betaines, amphoacetates or amphodiacetates like for example cocoamphodiacetate, Cocamide MEA or -DEA. Non-inoic surfactants are for example alkylglycosides and alkoxyglycosides.

Examples for bactericides are organic acids like formic acid, sorbic acid and benzoic acid. In addition esters of p-hydroxybenzoic acid, formaldehyde-releasing agents like DMDM hydantoin, imidazolidinylurea or methyl chloroisothiazolinone, methylisothiazolinone, dibromodicyanobutane, iodopropynyl butylcarbamte, phenoxyethanol or benzal alcohol can be used as bactericides.

Further useful pharmaceutically/dermatologically acceptable excipients may be inorganic or organic substances for topical administration.

Preferred pharmaceutically/dermatologically acceptable excipients are selected from the group consisting of solvents, gelling agents, buffers, surfactants, detergents, oils, alcohols, emulsifiers, solubilizers, humectants, fillers and bioadhesives.

Examples of particularly preferred excipients are water, plant oils, benzyl alcohols, polyethylene alcohols/glycols, gelatine, soya, carbohydrates (such as lactose or starch), lecithin, glycerol triacetate and other fatty acid glycerides, talc and cellulose. Examples of gelling agents as suitable excipients are natural gelling agents, such as pectin, agarose, gelatine and casein, or modified natural gelling agents, such as methyl cellulose, hydroxymethyl cellulose, hydroxymethylpropyl cellulose and carboxymethyl cellulose or full synthetic gelling agents, such as polyvinylalcohols, poly(meth)acrylacids, polyacrylamide, polyvinylpyrrolidone polypropylene glycol and polyethylene glycol.

The pH value of the formulation can be stabilized using buffer systems consisting of polyacids and their salts. Examples for such polyacids are citric acid, tartaric acid and malic acid.

Any further suitable pharmaceutical excipients known to the skilled person may preferably be added such as colorants.

Pharmaceutical compositions comprising eprotirome as used according to the present invention may be formulated in any of a variety of forms suitable, for example, for oral, topical or parenteral administration. Out of these, topical administration is preferred.

If eprotirome is systemically administered (parenteral administration) the amount is in a range of 0.01 to 3000 mg, more preferably from 0.05 mg to 1000 mg, more preferably from 0.1 to 100 mg per day.

If eprotirome is topically administered the amount is in a range of 0.01 mg to 1000 mg, more preferably from 0.05 mg to 100 mg, more preferably from 0.1 mg to 10 mg per day.

The composition preferably contains from about 0.0001% to about 10% (w/v), more preferably from about 0.001 % to about 5 % (w/v), most preferably from about 0.1 % to about 1% (w/v) of eprotirome, based on the total composition. If the amount is below the above values, the treatment and/or prevention is ineffective. On the other hand, if the amount is above the values mentioned, skin irritation may be observed.

Preferably, eprotirome for use according to the present invention as well as the pharmaceutical composition for use according the present invention can be co-administered with an additional therapeutically active substance. Preferably, these additional therapeutically active substances are chosen from a wide variety of molecules which can function in different ways to enhance hair growth effects of eprotirome (see, for example, www.regrowth.com for a listing of hair growth treatments). Particular classes of activity enhancers suitable for the co-administration with eprotirome as well as with compositions for use according to the present invention include hair growth stimulants. Non-limiting examples of agents that stimulate hair growth and/or arrest hair loss which may additionally be used in the compositions described herein, including both systemic and topical compositions, comprise, for example, benzalkonium chloride, benzethonium chloride, phenol, estradiol, diphenhydramine hydrochloride, caffeine, chlorpheniramine maleate, chlorophyllin derivatives, cholesterol, salicylic acid, arginine, cysteine, fatty acids, methionine, red pepper tincture, benzyl nicotinate, D,L-menthol, peppermint oil, calcium pantothenate, panthenol, castor oil, hinokitiol, prednisolone, resorcinol, monosaccharides and esterified monosaccharides, chemical activators of protein kinase C enzymes, glycosaminoglycan chain cellular uptake inhibitors, inhibitors of glycosidase activity, glycosaminoglycanase inhibitors, esters of pyroglutamic acid, hexosaccharic acids or acylated hexosaccharic acids, aryl-substituted ethylenes, N-acylated amino acids, tretinoin, cyclosporins, such as cyclosporin A, potassium channel blockers, such as minoxidil, 5-α-reductase inhibitors, such as finasteride or dutasteride, and androgen receptor antagonists, such as cyproterone acetate and ketoconazole.

Preferred additional therapeutically active substances (hair growth stimulants) to be added to the compositions for use accrding to the present invention are, for example, caffeine, minoxidil, cyproterone acetate and finasteride with minoxidil being most preferred.

Preferably, eprotirome of the present invention as well as the pharmaceutical composition for use according to the present invention comprising eprotirome can be co-administered with an anti-graying enhancer. Such an enchancer enhances anti-graying function.

Anti-graying enhancer for use according to the present invention can be chosen from the classes of substances stimulating the natural pigmentation process and melanisation.

Preferably, the anti-graying enhancer can be obtained from plants of the genus Echinacea. According to the present invention, plant has to be understood as the plant itself, its plant parts, extracts and pressed juice of Echinacea plants.

In particular, Echinacea plants can be selected from Echinacea angustifolia DC, Echinacea paradoxa (Norton), Echinacea simulata, E. atrorubens, E. tennesiensis, Echinacea strigosa (MCGREGOR), Echinacea laevigata, Echinacea purpurea (L.) Moench and Echinacea pallida (Nutt), and from these extracts and press juices are obtained.

Particularly preferred as anti-graying enhancer are extracts from Echinacea, more preferably extracts from Echinacea purpurea.

Moreover, eprotirome for use according to the present invention as well as the pharmaceutical composition for use according to the present invention comprising eprotirome preferably can be co-administered with a penetration enhancer.

Preferably, the penetration enhancer is selected from the group consisting of fatty alcohols, fatty acid esters, fatty acids, amides and amines, solvents, lecithin, bisabolol, 1,8 cineole, dimethyl isosorbide, menthol, terpenes, N-methyl-2-pyrrolidone, decylmethyl sulfoxide, dimethyl sulfoxide, 1-dodecyl azabicycloheptane-2-one, and N-dodecyl-2-pyrrolidone.

Preferred penetration enhancers are oleyl alcohol, diisopropyl sebacate, isopropyl myristate, oleyl oleate, oleic acid, ethomeen S12, dimethyl acetamide, propylene glycol, lecithin, bisabolol, 1,8 cineole, dimethyl isosorbide , menthol, terpenes, N-methyl-2-pyrrolidone, decylmethyl sulfoxide, dimethyl sulfoxide, 1-dodecyl azabicycloheptane-2-one, and N-dodecyl-2-pyrrolidone, particularly preferred are isopropyl myristate, propylene glycol and dimethyl isosorbide.

Preferably, these penetration enhancers are used in a concentration from 0.0001% to 10% (w/v), more preferably from about 0.001 % to about 5 % (w/v), most preferably from about 0.01 % to about 2 % (w/v), based on the total composition.

The combination of eprotirome and penetration enhancers results in an increased concentration of eprotirome in hair follicles. Therefore, a smaller amont of eprotirome can be administered in the treatment of the above-mentioned hair disorders in order to achieve a similar pharmacological efficiency. This results in a decreased systemic effect of eprotirome leading to a decreased risk of the occurrence of side effects in the patient.

A preferred pharmaceutical composition useful according to the present invention for topical administration includes
a) from 0.1 % to 1 % (w/v) eprotirome
b) from 30 % to 70 % (w/v) alcohol, e.g. ethanol
c) from 10 % to 30 % (w/v) synthetic gelling agent, e.g.polyacrylic acid
d) from 10 to 30 % (w/v) penetration enhancer, e.g. isopropyl myristate
e) q.s. water,
   based on the total composition.

A further preferred pharmaceutical composition useful according to the present invention for topical administration includes
a) from 0.1 % to 1 % (w/v) eprotirome
b) from 5 to 40 % (w/v) of a surfactant, e.g. ammonium lauryl sulphate and ammonium laureth sulfate
c) from 0.5 to 5 % (w/v) of a foaming agent, e.g. cocamide MEA
d) from 0.5 to 5 % (w/v) of a pearlizing agent, e.g.ethylene glycol distearate
e) from 0.5 to 5 % (w/v) of a thickening agent, e.g. cetyl alcohol
f) from 0.1 to 3 % (w/v) of a hair conditioning , e.g. polyquaternium-4
g) from 0.1 to 3 of a moisturizing agent, e.g.glycerin
h) from 10 to 30 % (w/v) penetration enhancer, e.g. dimethyl isosorbide
i) optionally from 5 to 10 % (w/v) further ingredients
j) q.s. water,
based on the total composition.

Preferably, the pharmaceutical composition for use according to the present invention can be used in patients whose hair disorders have failed to respond to other currently used treatments.

### Brief description of the drawings

### Treatment with eprotirome:

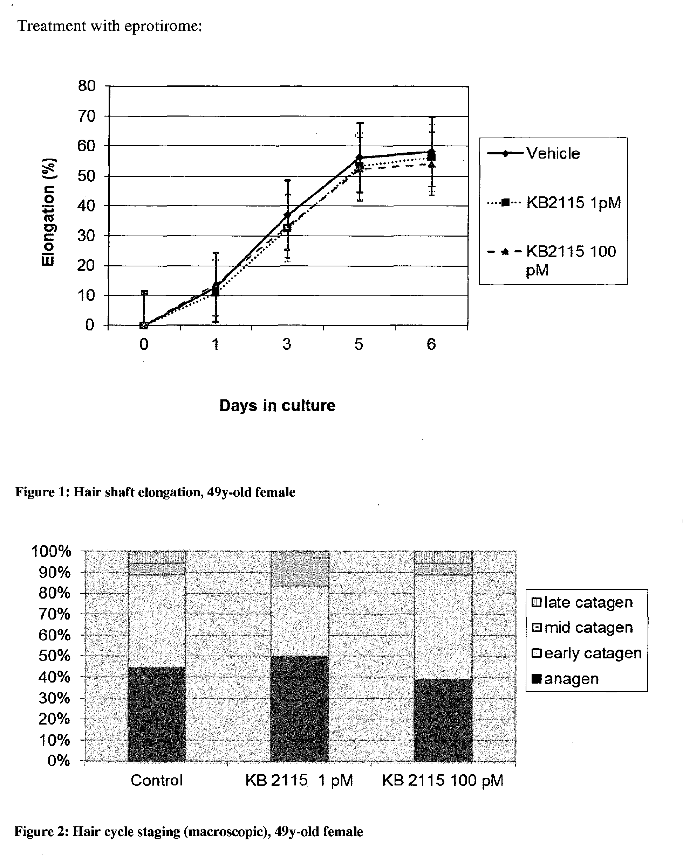

Figure 1 is a diagram that shows hair shaft elongation in hair follicles obtained from a 49 year-old female.
Figure 2 is a diagram that shows hair cycle staging (macroscopic) in hair follicles obtained from a 49 year-old female.

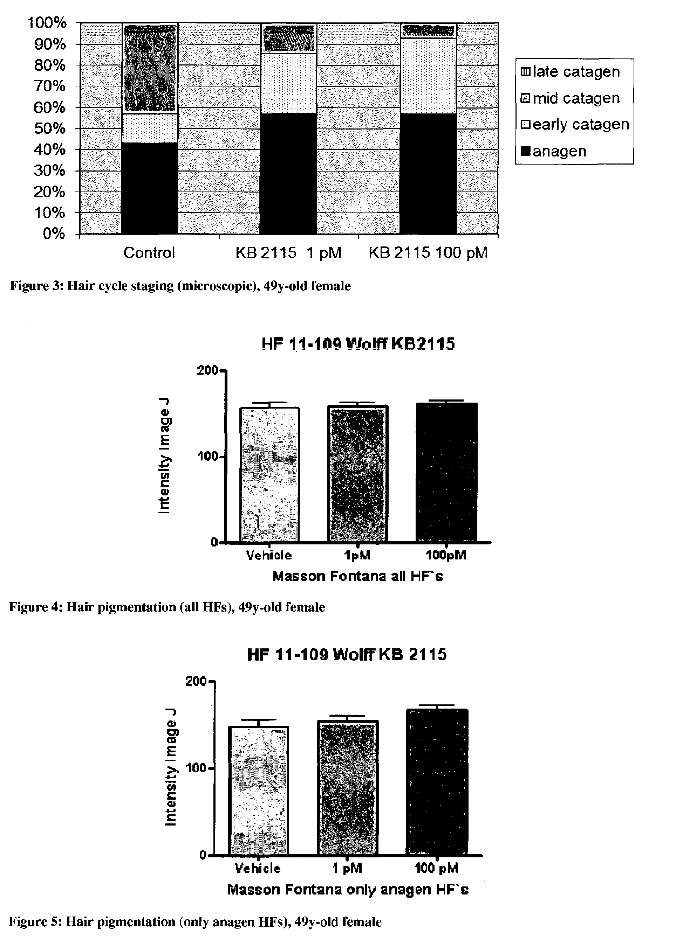

Figure 3 is a diagram that shows hair cycle staging (microscopic) in hair follicles obtained from a 49 year-old female.
Figure 4 shows hair pigmentation of all treated hair follicles from a 49 year-old female donor.
Figure 5 shows hair pigmentation of all treated hair follicles in the anagen phase from a 49 year-old female donor.

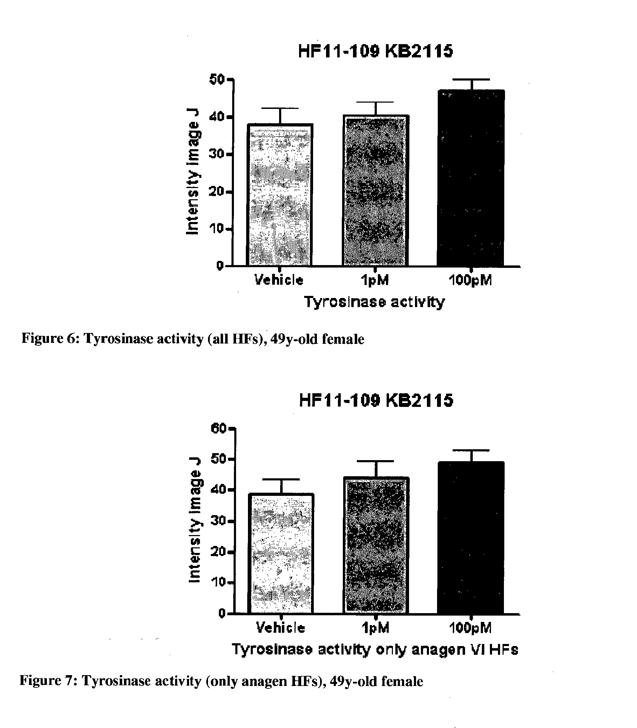

Figure 6 shows the upregulation of tyrosinase activity of hair follicles (all HFs) from a 49 year-old female donor.
Figure 7 shows the upregulation of tyrosinase activity of hair follicles (only anagen HFs) from a 49 year-old female donor.

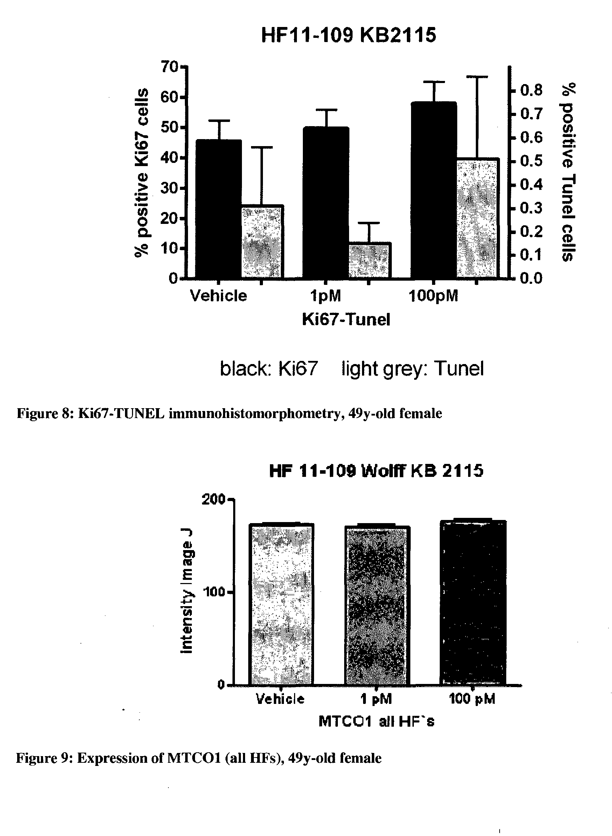

Figure 8 shows Ki-67 and TUNEL staining of treated hair follicles from a 49 year-old female donor.
Figure 9 shows the expression of MTCO1 in hair follicles (all HFs) from a 49 year-old female donor.

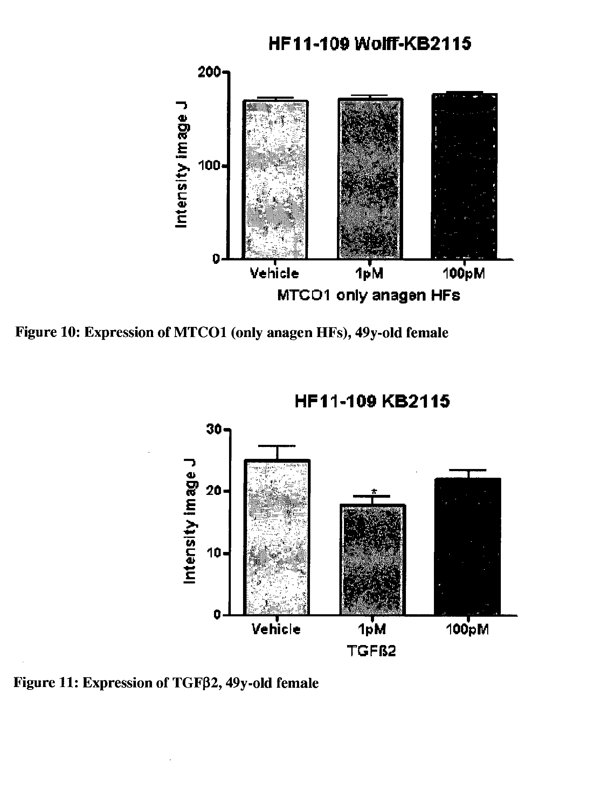

Figure 10 shows the expression of MTCO1 in hair follicles (only anagen HFs) from a 49 year-old female donor.
Figure 11 shows the reduced expression of TGFβ2 in treated hair follicles from a 49 year-old female donor.

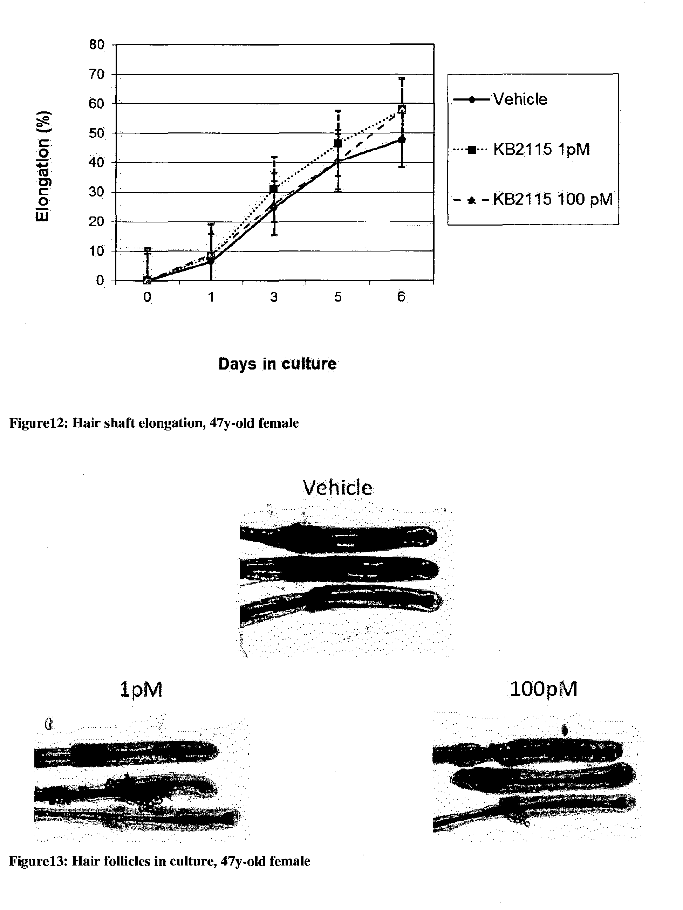

Figure 12 is a diagram that shows hair shaft elongation in hair follicles obtained from a 47 year-old female.
Figure 13 shows hair follicles in culture obtained from a 47 year-old female.

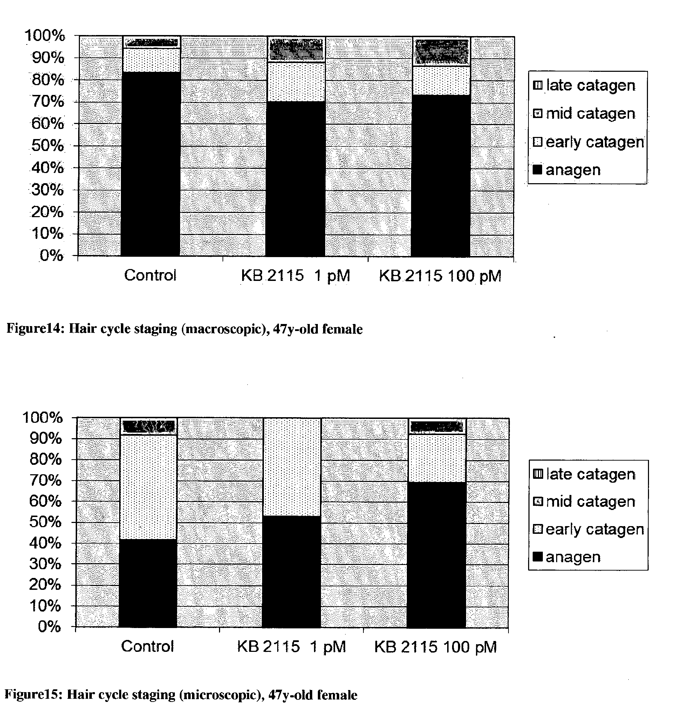

Figure 14 is a diagram that shows hair cycle staging (macroscopic) in hair follicles obtained from a 47 year-old female.
Figure 15 is a diagram that shows hair cycle staging (microscopic) in hair follicles obtained from a 47 year-old female.

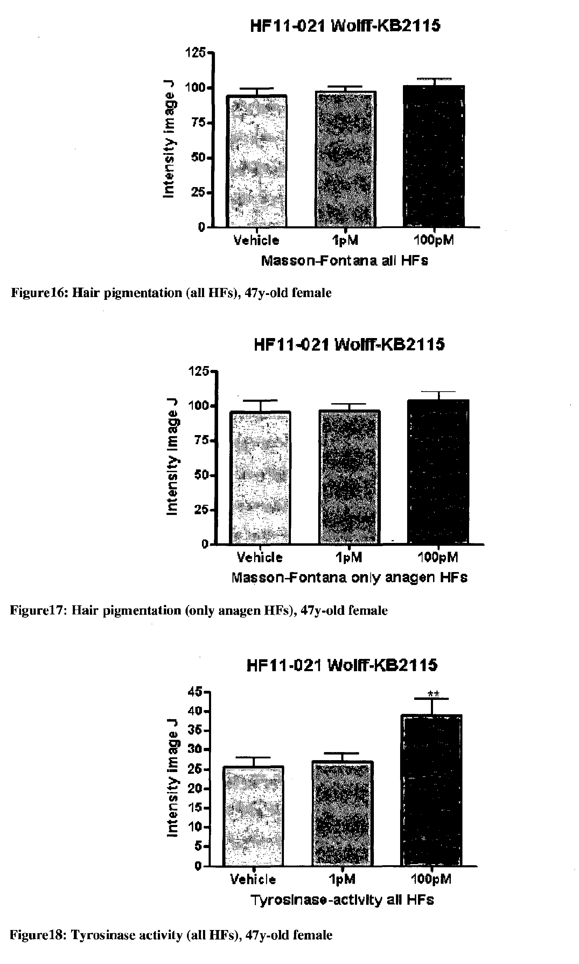

Figure 16 shows hair pigmentation of all treated hair follicles from a 47 year-old female donor.
Figure 17 shows hair pigmentation of all treated hair follicles in the anagen phase from a 47 year-old female donor.
Figure 18 shows the upregulation of tyrosinase activity of hair follicles (all HFs) from a 47 year-old female donor.

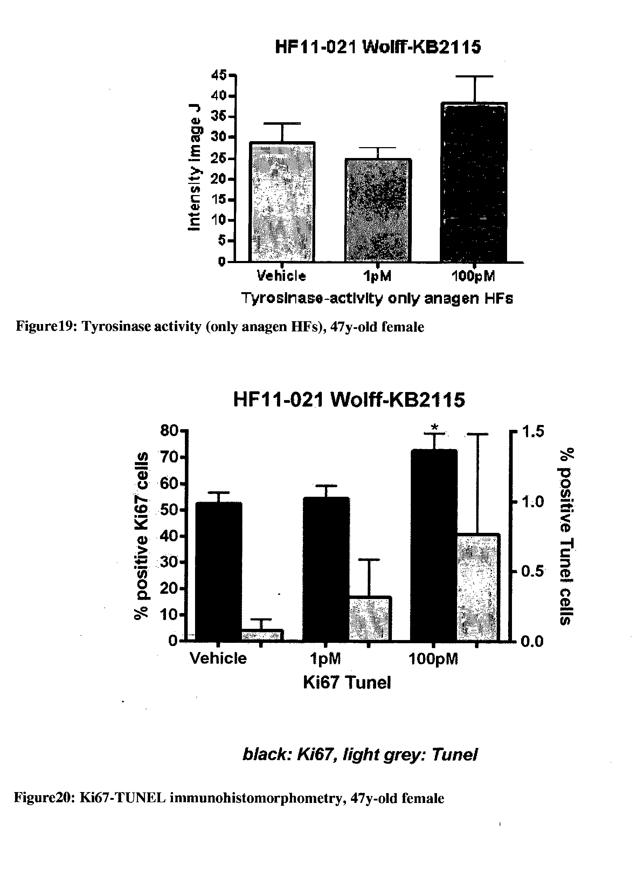

Figure 19 shows the upregulation of tyrosinase activity of hair follicles (only anagen HFs) from a 47 year-old female donor.
Figure 20 shows Ki-67 and TUNEL staining of treated hair follicles from a 47 year-old female donor.

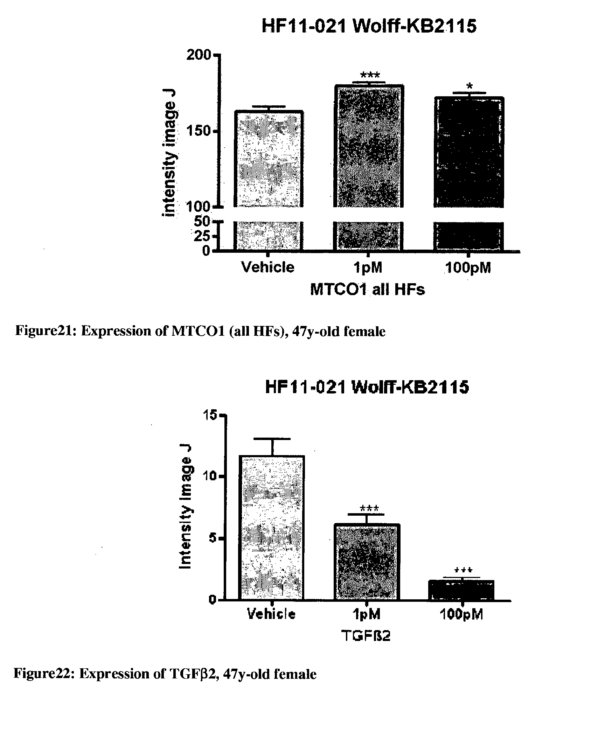

Figure 21 shows the upregulation of MTCO1 in treated hair follicles from a 47 year-old female donor.
Figure 22 shows the reduced expression of TGFβ2 in treated hair follicles from a 47 year-old female donor.
   Treatment with TRIAC:

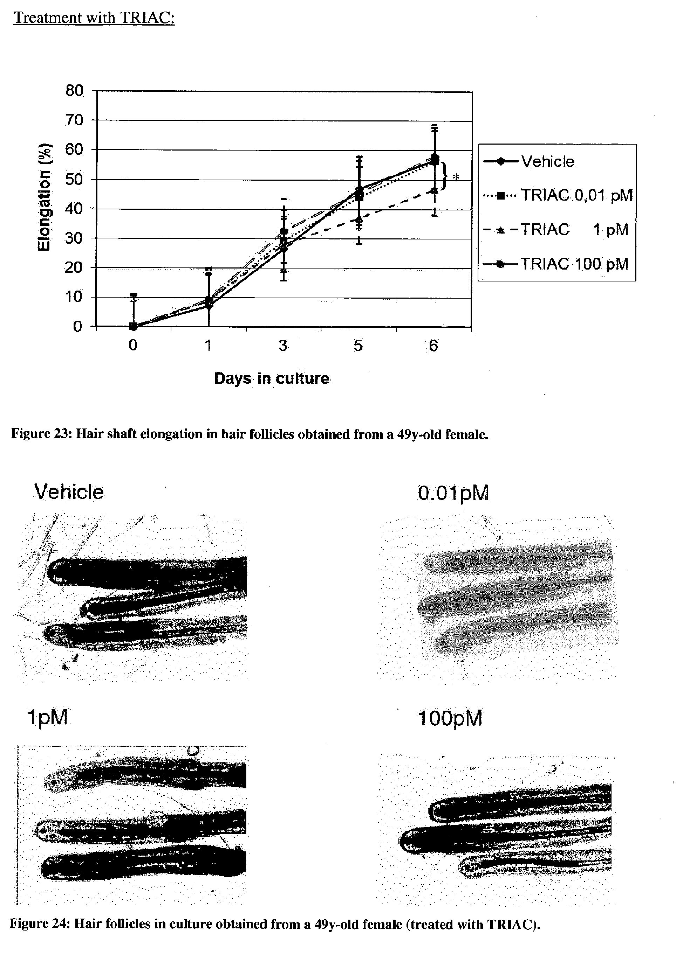

Figure 23 is a diagram that shows hair shaft elongation in hair follicles obtained from a 49 year-old female.
Figure 24 shows hair follicles in culture obtained from a 49 year-old female.

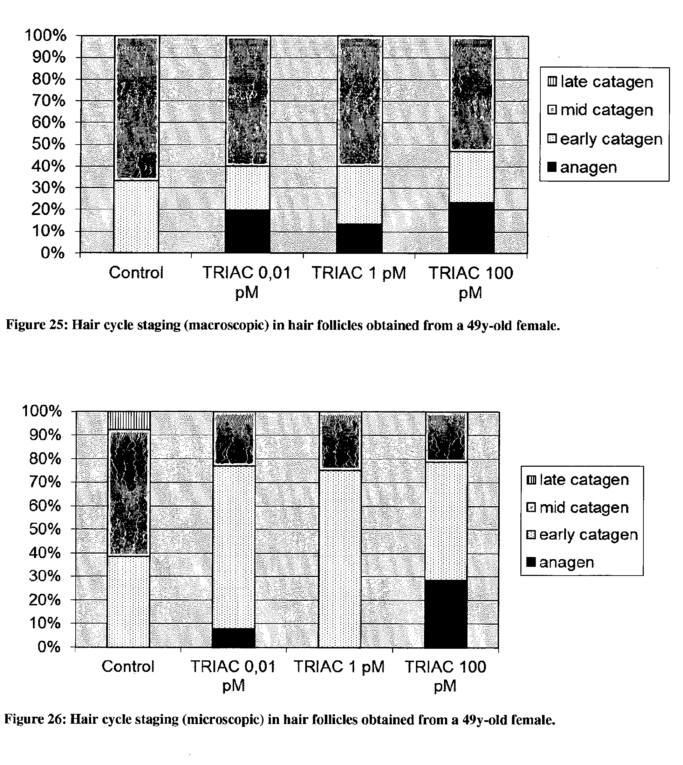

Figure 25 is a diagram that shows hair cycle staging (macroscopic) in hair follicles obtained from a 49 year-old female.
Figure 26 is a diagram that shows hair cycle staging (microscopic) in hair follicles obtained from a 49 year-old female.

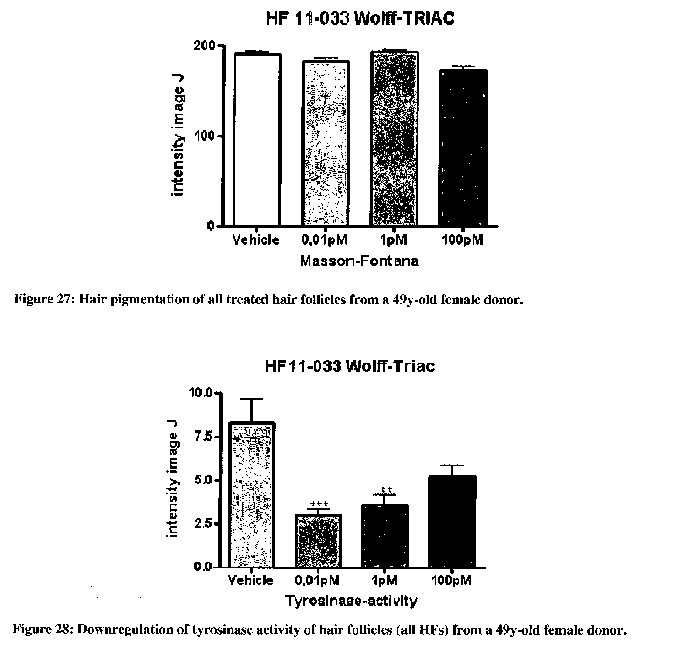

Figure 27 shows hair pigmentation of all treated hair follicles from a 49y-old female donor.
Figure 28 shows the downregulation of tyrosinase activity of hair follicles (all HFs) from a 49 year-old female donor.

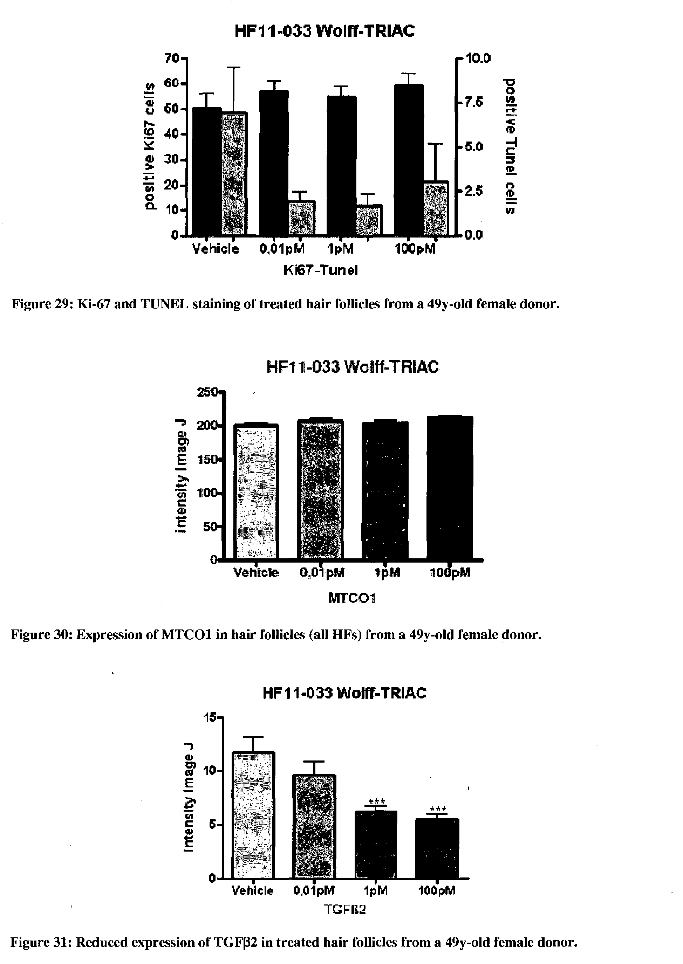

Figure 29 shows Ki-67 and TUNEL staining of treated hair follicles from a 49 year-old female donor.
Figure 30 shows the expression of MTCO1 in hair follicles (all HFs) from a 49 year-old female donor.
Figure 31 shows the reduced expression of TGFβ2 in treated hair follicles from a 49 year-old female donor.
   Treatment with PF-277343:

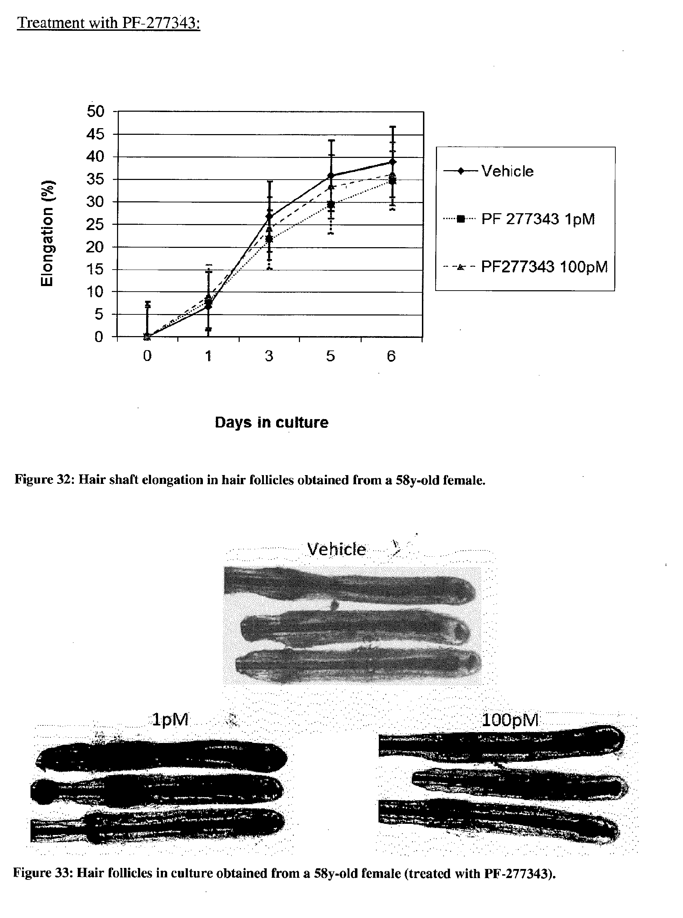

Figure 32 is a diagram that shows hair shaft elongation in hair follicles obtained from a 58 year-old female.
Figure 33 shows hair follicles in culture obtained from a 58 year-old female.

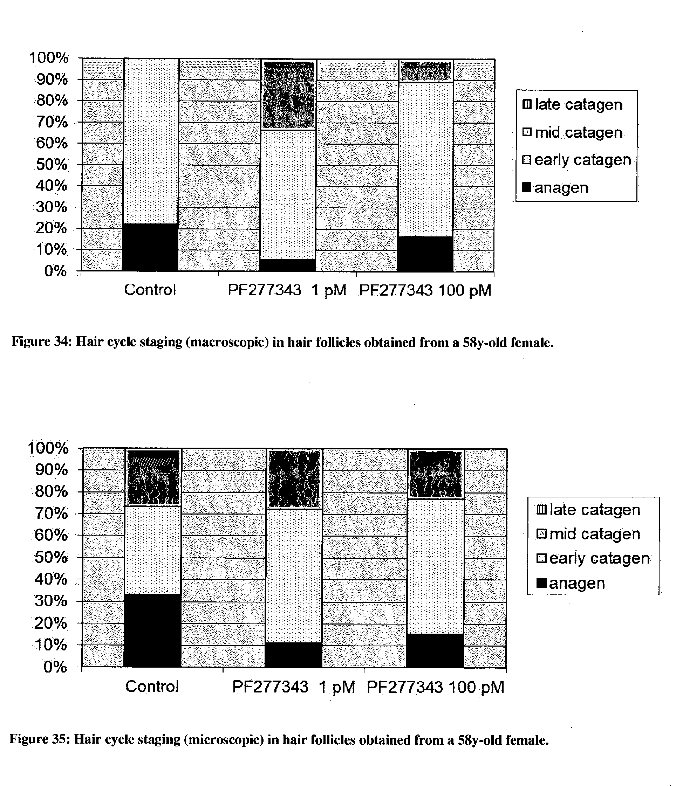

Figure 34 is a diagram that shows hair cycle staging (macroscopic) in hair follicles obtained from a 58 year-old female.
Figure 35 is a diagram that shows hair cycle staging (microscopic) in hair follicles obtained from a 58 year-old female.

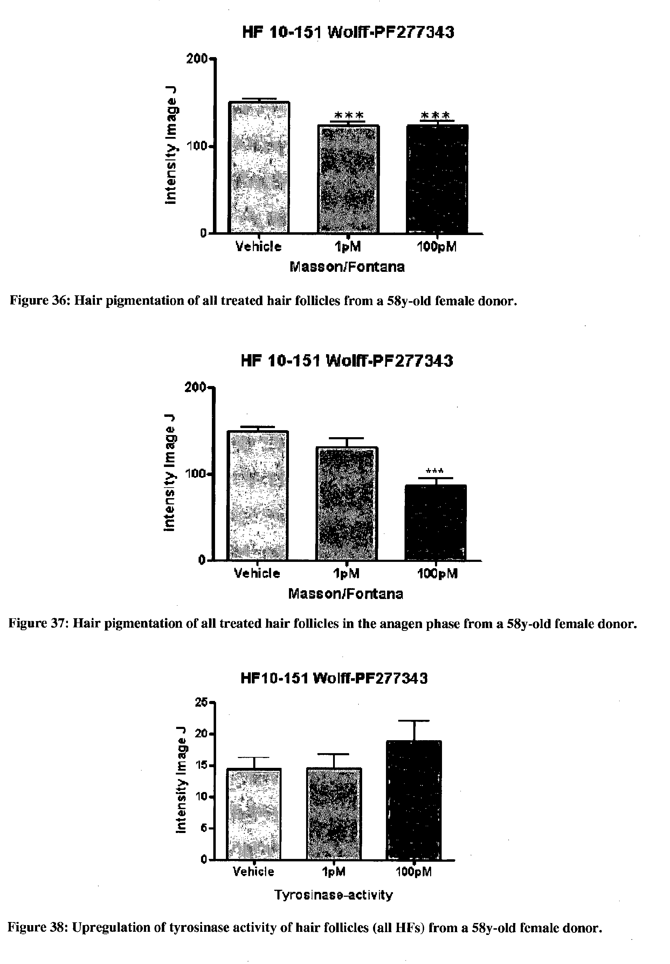

Figure 36 shows hair pigmentation of all treated hair follicles from a 58 year-old female donor.
Figure 37 shows hair pigmentation of all treated hair follicles in the anagen phase from a 58 year-old female donor.
Figure 38 shows the upregulation of tyrosinase activity of hair follicles (all HFs) from a 58 year-old female donor.

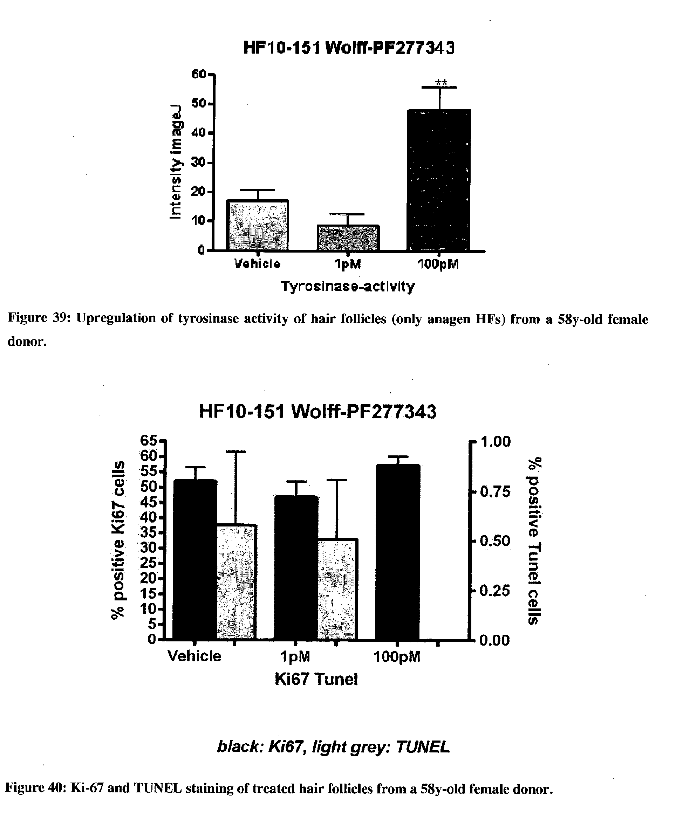

Figure 39 shows the upregulation of tyrosinase activity of hair follicles (only anagen HFs) from a 58 year-old female donor.
Figure 40 shows Ki-67 and TUNEL staining of treated hair follicles from a 58 year-old female donor.

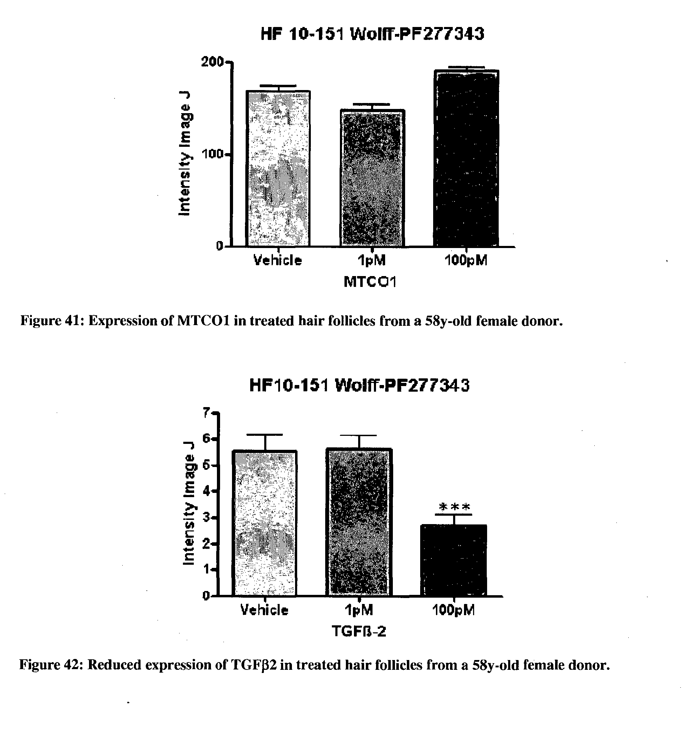

Figure 41 shows the expression of MTCO1 in treated hair follicles from a 58 year-old female donor.
Figure 42 shows the reduced expression of TGFβ2 in treated hair follicles from a 58 year-old female donor.

### EXAMPLES

### In vitro testing of thyroid hormone receptor agonists in human hair follicle organ culture

### A) Experimental Methods

### Tissue specimen:

Normal human scalp skin was obtained from women undergoing routine face-lift surgery after informed consent.

### Human hair follicle organ culture:

Test system: Philpott model (Philpott et al., J. Cell. Mol. Life Sci., 1990, 97: 463-471)

Mircodissected anagen VI hair follicles in 3 or 4 groups (3 HFs/well) were cultured at 6 days in a 24-well plate with 500 µl Williams E medium (Biochrom) supplemented with 100 IU/ml penicillin, 10 µg/ml streptomycin (Gibco), 10 µg/ml insulin (Sigma), 10 ng/ml hydrocortisone (Sigma) and 2 mmol/l L-glutamine (Invitrogen).

Eprotirome, TRIAC, PF-277343 (1 pM, 100 pM) or vehicle were administered once for each change of medium (i.e. every 48 h).

Anagen HFs and HFs were immediately embedded in Shandon Cryomatrix and snap frozen in liquid nitrogen. Six longitudinal sections of HFs were processed for immunohistological stainings.

### Hair shaft elongation:

Hair shaft length measurements of HFs were performed every second day on individual HFs using a Zeiss inverted binocular microscope with an eyepiece measuring graticule.

### HF cycle staging:

HF cycle staging was carried out according to defined morphological criteria, and the percentage of HFs in anagen and early, mid, or late catagen was determined.

### Hair pigmentation:

For histochemical visualization of melanin, Masson-Fontana staining was performed on frozen sections. Melanin was stained as brown dots and the degree of pigmentation was assessed by quantitative Masson-Fontana as described (Ito et al., Br. J. Dermatol., 2005, 152: 623-631). This method is a very sensitive and reliable indicator of changes in melanin synthesis, as shown by standard tyrosinase expression and enzyme activity assays (Kauser et al., FASEB J. 2006, 20:882-95). Staining intensity was analyzed in a defined reference region of the HF pigmentary unit and, using the ImageJ software (National Institute of Health).

### Proliferation and apoptosis measurements:

To evaluate apoptotic cells in co-localization with a proliferation marker Ki-67, a Ki-67/terminal dUTP nick-end labelling (TUNEL) double-staining method was used. Cryostat sections were fixed in paraformaldehyde and ethanol-acetic acid (2:1) and labelled with a digoxigenin-deoxy-UTP (ApopTag fluorescein in situ apoptosis detection kit; Intergen, Purchase, NY) in the presence of terminal deoxynucleotidyl transferase, followed by incubation with a mouse anti-Ki-67 antiserum (1:20 in PBS overnight at 4°C; Dako, Glostrup, Denmark). TUNEL-positive cells were visualized by an antidigoxigenin fluorescein isothiocyanate-conjugated antibody (ApopTag kit), whereas Ki-67 was detected by a rhodamine-labelled goat antimouse antibody (Jackson ImmunoResearch, West Grove, PA). Negative controls were performed by omitting terminal deoxynucleotidyl transferase and the Ki-67 antibody. Counterstaining was performed with 4',6-diamidino-2-phenylindole (DAPI) (Roche Molecular Biochemicals GmbH, Mannheim, Germany). Quantitative immunohistomorphometry was performed; Ki-67-, TUNEL-, or DAPI-positive cells were counted in a previously defined reference region of the HF matrix and epidermis, and the percentage of Ki-67/TUNEL-positive cells was determined.

### Statistical analysis:

Statistical analysis was performed using a two-tailed Student's t-test for unpaired samples.

### Example 1: Occipital scalp hair follicles from 49 year old female treated with eprotirome (KB2115).

Treatment with eprotirome did not significantly affect hair shaft elongation.
Eprotirome significantly increased the number of anagen and decreased the number of catagen HFs after 6 days, macroscopically and microscopically.

A slightly increased melanisation was observed for the 100 pM concentration in both analyzed groups (anagen HFs and all HFs).

Tyrosinase activity was slightly upregulated by 100 pM eprotirome (anagen HFs and all HFs). In line with the anagen-prolonging effect, the proliferation (Ki-67) of hair matrix keratinocytes is tendentially upregulated while the apoptosis (TUNEL) reveal no change in the treated group.

There was no significant change in the MTCO1 protein expression. Eprotirome significantly reduced TGFβ2 immunoreactivity in the 1 pM concentration group.

### Example 2: Occipital scaly hair follicles from 47 year old female treated with eprotirome (KB2115).

Eprotirome in both doses slightly stimulated hair shaft elongation. Macroscopically, eprotirome had no major effect on the duration of anagen. However, hair cycle histomorphometry reveals a clear anagen-prolonging effect of eprotirome.

Tendentially, 100 pM eprotirome slightly increased the melanin content (anagen HFs and all HFs). Tendentially, 100 pM eprotirome slightly increased the melanin content (all HFs). If only anagen HFs were analyzed, the effect was significant. Eprotirome (100 pM) slightly increased the melanin content (anagen HFs and all HFs).

Tyrosinase activity was upregulated in the 100 pM group (significant, if all HFs were analyzed). The high dose eprotirome (100 pM) treatment caused increased proliferation of the hair matrix keratinocytes (Ki-67). Eprotirome significantly up-regulates MTCO1 immunoreactivity. Eprotirome significantly reduced TGFβ2 immunoreactivity. The strongest effect was seen with the 100 pM concentration.

### Comparative example 1: Occipital scalp hair follicles from 49 year old female treated with TRIAC (triiodothyroacetic acid, Tiratricol).

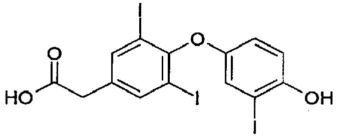

Treatment with TRIAC shows a slight inhibition of hair shaft elongation. A significant effect was observed for the 1 pM concentration. TRIAC prolonged anagen and inhibited catagen development in HFs after 6 days, macroscopically and microscopically. No significant effects on hair follicle melanin content were observerd (all HFs).

Tyrosinase activity was downregulated by TRIAC (all HFs). The effect was significant for the 0.1 pM and 1 pM groups.

In line with the anagen-prolonging effect, the proliferation (Ki-67) of hair matrix keratinocytes was slightly upregulated (not significant) and apoptosis (TUNEL) was inhibited (not significant).

There was no significant change in the MTCO1 protein expression. TRIAC reduced TGFβ2 immunoreactivity in all concentrations. A significant effect was seen for the 1 pM and 100 pM concentration groups. However, TRIAC showed low stability and an inferior penetration in human skin.

### Comparative example 2: Occipital scalp hair follicles from 58 year old female treated with PF-277343.

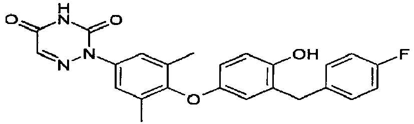

Treatment with 1 pM showed a slight (not significant) tendency towards inhibition of hair shaft elongation.

Macroscopic HF assessment suggests that the 1 pM dose of PF-277343 prematurely induces catagen. In line with the macroscopic staging, quantitative hair cycle histomorphometry confirmed that PF-277343 promoted premature catagen development at 1 pM and 100 pM.

A significantly decreased melanisation was observed for the 1 pM and 100 pM concentration when all HFs were analyzed. When only anagen HFs were analysed a strong reduction of melanin production was seen for the 100 pM group (significant). The reduction in melanisation independently confirms the catagen-promoting effect.

Tyrosinase activity was slightly upregulated by 100 pM PF-277343 (anagen HFs and all HFs). This effect was significant, if only anagen HFs were analysed.

PF-277343 did not show effects on the proliferation (Ki-67) of hair matrix keratinocytes. In the 100 pM concentration is apoptosis (TUNEL) is slightly inhibited (not significant). There was no significant change in the MTCO1 protein expression.

PF-277343 significantly reduced TGFβ2 immunoreactivity in the 100 pM concentration group.

Although eprotirome, TRIAC and PF-277343 belong to the same class of compounds, namely thyroid hormone receptor agonists, the three compounds surprisingly exhibited a different response pattern when applied to human scalp hair follicles.

Macroscopic and microscopic analysis of hair cycle staging revealed that only eprotirome and TRIAC were able to prolong anagen and to inhibit catagen development. In contrast treatment with PF-277343 caused hair follicles to enter the catagen phase prematurely, an effect which is not desired for agents that should arrest hair loss or promote hair growth. In line with this finding is the fact that there was a tendency of a slight inhibition of hair shaft elongation observed for PF-277343.

Surprisingly, eprotirome also exhibited positive effects on pigmentation of hair follicles as shown by upregulation of tyrosinase activity and increased melanin content of treated hair follicles. In contrast no effects on melanin content were observed for hair follicles treated with TRIAC and negative effects, i.e. a decrease in melanin content, was observed for hair follicles treated with PF-277343. This reduced melanisation could lead to side effects such as greying.

Thus, the overall profile of eprotirome is favorable, since it has a positive effect on hair cycle staging and melanisation.

### Examples for compositions/formulations useful according to the present invention

Composition for topical administration: (in % (w/v) based on the total compositon)

| | |
|---|---|
| Eprotirome | 1% |
| Ethanol | 61% |
| Propylene glycol | 19% |
| Dimethyl isosorbide | 19% |

### Shampoo 1: (in % (w/v) based on the total compositon)

| | |
|---|---|
| Eprotirome | 0.2% |
| Ammonium lauryl sulfate | 11.5% |
| Ammonium laureth sulfate | 4% |
| Cocamide MEA | 2% |
| Ethylene glycol distearate | 2% |
| Cetyl alcohol | 2% |
| Stearyl alcohol | 1.2% |
| Glycerin | 1% |
| Polyquaternium 10 | 0.5% |
| Sodium Chloride | 0.1% |
| Sucrose polyesters of cottonate fatty acid | 3% |
| Sucrose polyesters of behenate fatty acid | 2% |
| Lauryl dimethyl amine oxide | 1.5% |
| DMDM hydantoin | 0.15% |
| Phenoxyethanol | 0.5% |
| Fragrance | 0.5% |
| Water | q.s. |

### Shampoo 2: (in % (w/v) based on the total compositon)

| | |
|---|---|
| Eprotirome | 0.3% |
| Sodium laureth sulfate | 10% |
| Cocoamphoacetate | 2% |
| Glycerin | 2% |
| Panthenol | 0.5% |

| | |
|---|---|
| Hydrolyzed collagen | 1.0% |
| Sodium polyphosphate | 0.1% |
| Fragrance | 0.3% |
| Sodium chloride | 0.3% |
| Piroctone olaminc | 0.5% |
| Formic acid | 0.2% |
| Water | q.s. |

### Shampoo 3: (in % (w/v) based on the total compositon)

| | |
|---|---|
| Eprotirome | 0.5% |
| Sodium lauryl sulfate | 4% |
| Sodium laureth sulfate | 5% |
| Hydroxypropyltrimonium hydrolyzed wheat protein | 1% |
| Disodium laureth sulfosuccinate | 1% |
| Biotin | 0.02% |
| Tocopherylacetate | 0.3% |
| Citric acid | 0.2% |
| Fragrance | 0.3% |
| Potassium sorbate | 0.2% |
| DMDM hydantoin | 0.1% |
| Water | q.s. |

## Claims

1. Eprotirome for use in the prevention and/or treatment of hair loss in a mammal.

2. Eprotirome for use according to claim 1 wherein the hair loss is selected from alopecia areata, androgenetic alopecia including male pattern and female pattern baldness and chemotherapy-induced hair loss.

3. Eprotirome for use according to any one of claims 1 to 2 wherein the mammal is a human being.

4. Eprotirome for use according to any one of claims 1 to 3 wherein eprotirome is applied topically on the skin of a mammal.

5. A pharmaceutical composition for use in the prevention and/or treatment of hair loss comprising:
(a) eprotirome as active ingredient, and
(b) a pharmaceutically acceptable excipient.

6. The pharmaceutical composition for use according to claim 5 wherein the hair loss is selected from alopecia areata, androgenetic alopecia including male pattern and female pattern baldness and chemotherapy-induced hair loss.

7. The pharmaceutical composition for use according to claim 5 and/or 6, further comprising (c) a penetration enhancer, preferably selected from the group of isopropyl myristate, propylene glycol and dimethyl isosorbide.

8. The pharmaceutical composition for use according to any one of claims 5 to 7, which comprises 0.0001 to about 10 wt. % of eprotirome, based on the total weight of the pharmaceutical composition.

9. The pharmaceutical composition for use according to any one of claims 5 to 8, wherein the pharmaceutically acceptable excipient is selected from the group consisting of solvents, gelling agents, buffers, surfactants, detergents, oils, alcohols, emulsifiers, solubilizers, humectants, fillers and bioadhesives.

10. The pharmaceutical composition for use according to any one of claims 5 to 9, further comprising an additional therapeutically active substance.

11. The pharmaceutical composition for use according to claim 10, wherein the additional therapeutically active substance is selected from caffeine, finasteride, minoxidil, cyproterone acetate.

12. The pharmaceutical composition for use according to any one of claims 5 to 11, further comprising an anti-graying enhancer.

13. The pharmaceutical composition for use according to claim 12, wherein the anti-graying enhancer is an extract of Echinacea.

14. Non-therapeutic use of eprotirome in the treatment and/or prevention of hair loss in mammals and/or in the prevention of hair conditions, preferably in the prevention and/or reversal of hair graying.

## Patentansprüche

1. Eprotirom zur Verwendung in der Vorbeugung und/oder Behandlung von Haarausfall in einem Säuger.

2. Eprotirom zur Verwendung nach Anspruch 1, worin der Haarausfall ausgewählt ist aus Alopecia areata, androgenetischer Alopezie einschließlich der Kahlheit nach männlichem Muster und nach weiblichem Muster sowie Chemotherapie-induziertem Haarausfall.

3. Eprotirom zur Verwendung nach einem der Ansprüche 1 bis 2, worin der Säuger ein Mensch ist.

4. Eprotirom zur Verwendung nach einem der Ansprüche 1 bis 3, worin Eprotirom auf die Haut eines Säugers topisch aufgetragen wird.

5. Pharmazeutische Zusammensetzung zur Verwendung in der Vorbeugung und/oder Behandlung von Haarausfall, umfassend:
(a) Eprotirom als Wirkstoff und
(b) einen pharmazeutisch annehmbaren Hilfsstoff.

6. Pharmazeutische Zusammensetzung zur Verwendung nach Anspruch 5, worin der Haarausfall ausgewählt ist aus Alopecia areata, androgenetischer Alopezie einschließlich der Kahlheit nach männlichem Muster und nach weiblichem Muster sowie Chemotherapie-induziertem Haarausfall.

7. Pharmazeutische Zusammensetzung zur Verwendung nach Anspruch 5 und/oder 6, weiterhin umfassend
(c) einen Penetrationsverstärker, vorzugsweise ausgewählt aus der Gruppe aus Isopropylmyristat, Propylenglykol und Dimethylisosorbid.

8. Pharmazeutische Zusammensetzung zur Verwendung nach einem der Ansprüche 5 bis 7, welche 0,0001 bis ungefähr 10 Gew.-% Eprotirom auf der Basis des Gesamtgewichts der pharmazeutischen Zusammensetzung umfasst.

9. Pharmazeutische Zusammensetzung zur Verwendung nach einem der Ansprüche 5 bis 8, worin der pharmazeutisch annehmbare Hilfsstoff ausgewählt ist aus der Gruppe, bestehend aus Lösungsmitteln, Geliermitteln, Puffern, Tensiden, Detergenzien, Ölen, Alkoholen, Emulgatoren, Solubilisatoren, Feuchthaltemitteln, Füllstoffen und Bioadhäsiven.

10. Pharmazeutische Zusammensetzung zur Verwendung nach einem der Ansprüche 5 bis 9, weiterhin umfassend eine zusätzliche therapeutisch wirksame Substanz.

11. Pharmazeutische Zusammensetzung zur Verwendung nach Anspruch 10, worin die zusätzliche therapeutisch wirksame Substanz ausgewählt ist aus Koffein, Finasterid, Minoxidil, Cyproteronacetat.

12. Pharmazeutische Zusammensetzung zur Verwendung nach einem der Ansprüche 5 bis 11, weiterhin umfassend einen Anti-Grau-Verstärker.

13. Pharmazeutische Zusammensetzung zur Verwendung nach Anspruch 12, worin der Anti-Grau-Verstärker ein Extrakt aus Echinacin ist.

14. Nicht-therapeutische Verwendung von Eprotirom in der Behandlung und/oder Vorbeugung von Haarausfall in Säugern und/oder in der Vorbeugung von Haarkonditionen, vorzugsweise in der Vorbeugung und/oder Umkehrung des Ergrauens von Haar.

## Revendications

1. Eprotirome pour son utilisation dans la prévention et/ou le traitement de la perte de cheveux chez un mammifère.

2. Eprotirome pour son utilisation selon la revendication 1, dans lequel la perte de cheveux est sélectionnée parmi la pelade, l'alopécie androgénétique comprenant la calvitie chez l'homme et la calvitie chez la femme et la perte de cheveux induite par une chimiothérapie.

3. Eprotirome pour son utilisation selon l'une quelconque des revendications 1 à 2, dans lequel le mammifère est un être humain.

4. Eprotirome pour son utilisation selon l'une quelconque des revendications 1 à 3, dans lequel l'éprotirome est appliqué par voie topique sur la peau d'un mammifère.

5. Composition pharmaceutique pour son utilisation dans la prévention et/ou le traitement de la perte de cheveux comprenant :
(a) l'éprotirome en tant qu'ingrédient actif, et
(b) un excipient pharmaceutiquement acceptable.

6. Composition pharmaceutique pour son utilisation selon la revendication 5, dans laquelle la perte de cheveux est sélectionnée parmi la pelade, l'alopécie androgénétique comprenant la calvitie chez l'homme et la calvitie chez la femme et la perte de cheveux induite par la chimiothérapie.

7. Composition pharmaceutique pour son utilisation selon la revendication 5 et/ou la revendication 6, comprenant en outre :
(c) un agent améliorant la pénétration, de préférence sélectionné dans le groupe constitué du myristate d'isopropyle, du propylène glycol et de l'isosorbide de diméthyle.

8. Composition pharmaceutique pour son utilisation selon l'une quelconque des revendications 5 à 7, qui comprend de 0,0001 à environ 10 % en poids d'éprotirome, sur la base du poids total de la composition pharmaceutique.

9. Composition pharmaceutique pour son utilisation selon l'une quelconque des revendications 5 à 8, dans laquelle l'excipient pharmaceutiquement actif est sélectionné dans le groupe constitué de solvants, d'agents de gélification, de tampons, de surfactants, de détergents, d'huiles, d'alcools, d'émulsifiants, de solubilisants, d'humectants, de charges et de bioadhésifs.

10. Composition pharmaceutique pour son utilisation selon l'une quelconque des revendications 5 à 9, comprenant en outre une substance thérapeutiquement active supplémentaire.

11. Composition pharmaceutique pour son utilisation selon la revendication 10, dans laquelle la substance thérapeutiquement active supplémentaire est sélectionnée parmi la caféine, le finastéride, le minoxidil, l'acétate de cyprotérone.

12. Composition pharmaceutique pour son utilisation selon l'une quelconque des revendications 5 à 11, comprenant en outre un activateur anti-grisonnement.

13. Composition pharmaceutique pour son utilisation selon la revendication 12, dans laquelle l'activateur anti-grisonnement est un extrait d'Echinacea.

14. Utilisation non thérapeutique d'éprotirome dans le traitement et/ou la prévention de la perte de cheveux chez les mammifères et/ou dans la prévention d'affections capillaires, de préférence dans la prévention et/ou l'inversement du grisonnement des cheveux.
